# EUROPEAN PATENT SPECIFICATION

(11) **EP 3 567 114 B1**
(45) Date of publication and mention of the grant of the patent: **03.03.2021**
(21) Application number: 19173044.9
(22) Date of filing: 07.05.2019
(51) Int. Cl.: C12P 3/00, C12N 1/20, A61K 36/02, A61K 35/00, C12N 1/12

(54) **PROCESS FOR THE SYNTHESIS OF SILVER NANOPARTICLES USING PROBIOTIC STRAINS GROWN IN ALGAE, TERNARY MIXTURE THUS OBTAINED AND USES THEREOF**
VERFAHREN ZUR SYNTHESE VON SILBERNANOPARTIKELN UNTER VERWENDUNG VON IN ALGEN GEWACHSENEN PROBIOTISCHEN STÄMMEN, DADURCH ERHÄLTLICHE DREIFACHMISCHUNGEN UND IHRE VERWENDUNG
PROCÉDÉ DE SYNTHÈSE DE NANOPARTICULES D'ARGENT À L'AIDE DE SOUCHES PROBIOTIQUES CULTIVÉES EN ALGUES, MÉLANGE TERNAIRE AINSI OBTENU ET UTILISATIONS DE CEUX-CI

(30) Priority: 07.05.2018 IT 201800005108
(43) Date of publication of application: 13.11.2019
(73) Proprietor: OCEAN FARMA S.r.l, 04100 Latina (IT)
(72) Inventor: LETER, Beatrice, I-00141 ROMA (IT); BRUSCHI, Raffaella, I-00063 Campagnano di Roma (RM) (IT)
(74) Representative: Calogero, Ida

(56) References cited:
- WO-A1-2008/003522
- WO-A1-2017/216818
- ZUZER H DHOONDIA ET AL: "Lactobacillus Mediated Synthesis of Silver Oxide Nanoparticles", NANOSYSTEMS, NANOMATERIALS, NANOTECHNOLOGIES, vol. 2, 20 December 2012 (2012-12-20), page 15, XP055531407, HR ISSN: 1847-9804, DOI: 10.5772/55741
- MAHDI MOHSENIAZAR ET AL: "Potential of Microalgae and Lactobacilli in Biosynthesis of Silver Nanoparticles", BIOIMPACTS, vol. 1, no. 3, 1 September 2011 (2011-09-01), pages 149-152, XP055530931, ISSN: 2228-5660
- AHMED SHAKEEL ET AL: "A review on plants extract mediated synthesis of silver nanoparticles for antimicrobial applications: A green expertise", JOURNAL OF ADVANCED RESEARCH, ELSEVIER, AMSTERDAM, NL, vol. 7, no. 1, 9 March 2015 (2015-03-09), pages 17-28, XP029371333, ISSN: 2090-1232, DOI: 10.1016/J.JARE.2015.02.007
- LIESJE SINTUBIN ET AL: "Lactic acid bacteria as reducing and capping agent for the fast and efficient production of silver nanoparticles", APPLIED MICROBIOLOGY AND BIOTECHNOLOGY, SPRINGER, BERLIN, DE, vol. 84, no. 4, 2 June 2009 (2009-06-02), pages 741-749, XP019737747, ISSN: 1432-0614, DOI: 10.1007/S00253-009-2032-6
- DEENDAYAL MANDAL ET AL: "The use of microorganisms for the formation of metal nanoparticles and their application", APPLIED MICROBIOLOGY AND BIOTECHNOLOGY, SPRINGER, BERLIN, DE, vol. 69, no. 5, 1 January 2006 (2006-01-01), pages 485-492, XP019332048, ISSN: 1432-0614, DOI: 10.1007/S00253-005-0179-3

## Description

The present invention relates to a process for the biogenic synthesis of silver nanoparticles by probiotic strains belonging to the genera *Lactobacillus sp.* and *Bifidobacterium sp.* grown in unicellular algae, the silver nanoparticles obtained from the fermentation process combined with probiotics, and their use in the medical field as anti-inflammatory and/or antiseptic agents.

The ability to aggregate silver ion in nanoparticles of different dimensions and quality seems to be a common and known characteristic for different microorganisms [1].

It has in fact already been described that silver nanoparticles can be synthesized from *Bacillus spp* [2]. In particular, silver nanoparticles can be synthesized from *Bacillus licheniformis* (*B. licheniformis*) starting from silver ions ([3,4]).

Furthermore, it has been demonstrated that *B. subtilis* produces silver nanoparticles in aqueous liquid medium irradiated with microwaves in order to increase the reaction rate and reduce the aggregation of the nanoparticles produced [5].

Silver nanoparticles of different compositions have been successfully synthesized by *Pseudomonas stutzeri* AG259 [6].

The synthesis of silver nanoparticles, obtained by reducing Ag⁺ aqueous ions by notroreductase enzymes, has been demonstrated in *Klebsiella pneumonia, E. coli* and *Enterobacter cloacae* [7]*.*

Monodispersed and stable silver nanoparticles have been synthesized by *Aeromonas sp.* SH10 and *Corynebacterium sp.* SH09 reducing [Ag(NH₃)₂]⁺ [8]. It was hypothesized that [Ag(NH₃)₂]⁺ reacted first with OH⁻ to form Ag₂O, then it was metabolized independently and reduced to silver nanoparticles.

It has also been reported that strains of *Lactobacillus* in the presence of silver ions have synthesized nanoparticles at the intracellular level [9, 10]. Among the lactobacilli, *Lactobacillus acidophilus, casei, fermentum, plantarum, rhamnosus* and *reuteri* are capable of synthesizing nanoparticles [9, 11,12].

Silver nanoparticles, characterized by spectroscopic methods, have been synthesized using extracts of the root of *Rheum palmatum* [13]. The average particle size was found to be 121 ± 2 nm with zeta potential values of -21.6 mv, measured with the dynamic light scattering method (DLS). The nanoparticles synthesized showed a significant antimicrobial activity, particularly against *Staphylococcus aureus* and *Pseudomonas aeruginosa,* with IC90 values equal to 15 mcg / ml and 7.5 mcg / ml, respectively.

The growth of micro-organisms in single-cell algae, on the other hand, is already known, as reported in international patent application WO 2006/112364 and in the publication of Talukder N.R. et al [14]. This study in turn reports that the extracts of unicellular algae *Hydrolictoyon reticulum* and *Nannochloropsis salina* had already been previously described as a growth medium for *Lactobacillus paracasei* [15].

The authors of the present invention have now developed a process for the biogenic synthesis of silver nanoparticles starting from ionic silver using probiotic strains belonging to the genera *Lactobacillus sp.* or *Bifidobacterius sp.,* wherein the growth of the above-mentioned microorganisms and the synthesis of these nanoparticles take place using, as culture medium, a unicellular alga under aerobic or anaerobic conditions, which allows a mixture of silver nanoparticles, probiotic strains and unicellular algae rich in omega-3 fatty acids to be obtained, with marked anti-inflammatory and/or antibacterial properties.

According to the present invention the Lactobacilli strains can be grown under aerobic conditions (the probiotics are grown in a flask with a cotton cap, therefore in the presence of atmospheric oxygen) or anaerobic conditions (the probiotics are grown in a flask with a sealed cap, therefore in the absence of oxygen), anaerobes being optional. If a strain of Bifidobacterium spp. is used, the growth necessarily takes place under conditions of anaereobiosis, anaerobes being obligatory.

An object of the present invention therefore relates to a process for the synthesis of a ternary mixture of silver nanoparticles, probiotic and unicellular alga rich in omega-3 fatty acids comprising the following steps:
a) inoculation of a probiotic strain belonging to the genus *Lactobacillus sp.* or *Bifidobacterium sp.* at a concentration ranging from 10⁵ to 10⁷ CFU/ml in an aqueous medium comprising unicellular algae rich in omega-3 fatty acids and an inorganic donor of silver ions;
b) cultivation at 37°C under aerobic or anaerobic conditions for 5-6 days.

The concentration of inoculum of the probiotic strain according to step a) is selected so as to ensure a high concentration of viable probiotic in the final ternary mixture, ranging from 10⁷ to 10⁹ CFU / ml.

The process described above can comprise a further optional step c) for the lyophilization of the mixture obtained in step b).

Contrary to the known industrial processes of use of unicellular algae, the process of the present invention does not provide any extraction step of the organic phase or enrichment of the lipid phase. Microalgae are in fact used in their entirety as the sugar and protein component is functional to the growth of the probiotic, whereas the lipid portion is maintained as a source of polyunsaturated omega-3 fatty acids within the final product.

According to the present invention, the Lactobacillus strains can be grown under aerobic conditions (the probiotics are grown in a flask with a cotton cap, therefore in the presence of atmospheric oxygen) or anaerobic conditions (the probiotics are grown in a flask with a sealed cap, therefore in the absence of oxygen) anaerobes being optional. If a strain of Bifidobacterium spp. is used, the growth necessarily takes place in conditions of anaereobiosis, anaerobes being obligatory.

According to a preferred embodiment of the above-mentioned fermentation processes, said unicellular algae are present in the culture medium at a concentration of 30-40 g/L.

The unicellular algae used in the process of the present invention are green or blue microalgae characterized in that they are rich in omega 3 and have no cellulosic wall. This makes their use possible as such without resorting to the subsequent homogenization step.

Said green or blue microalgae rich in omega-3 acids preferably belong to the species selected from the group consisting of *Pavlova sp.* (e.g. *Pavlova lutheri*), *Isochrisys sp.* (e.g. *Isochrysis T-iso), Nannochloropsis sp.* (e.g. *Nannochloropsis salina, Nannochloropsis gaditana, Nannochloropsis oculata), Porphyridium sp.* (e.g. *Porphyridium cruentum), Cryptomonas sp., Rhodomonas sp.* (e.g. *Rhodomonas salina), Tetraselmis sp.* (e.g. *Tetraselmis suecica*). According to a preferred embodiment, the microalga belongs to the species *Nannochloropsis gaditana.*

In a preferred embodiment of the fermentation process according to the invention, said inorganic donor of silver ions is added to the culture medium at a concentration ranging from 2 to 5 µM.

Said inorganic donor of silver ions is more preferably silver nitrate (AgNO₃).

The species of *Lactobacillus* that can be advantageously used for the synthesis of silver nanoparticles if grown in a medium containing unicellular algae are selected from the group consisting of *L. reuteri, L. brevis, L. buchneri, L. fermentum* (obligatory heterofermentative Lactobacilli) or *L. casei, L. curvatus, L. plantarum, L.sakei, L. paracasei* (optional heterofermentative Lactobacilli).

Alternatively, the species of *Bifidobacterium* that can be advantageously used for the synthesis of silver nanoparticles if grown in a medium containing unicellular algae are selected from the group consisting of *B. lactis* and *B. brevis.*

A further object of the present invention relates to a ternary mixture that can be obtained by means of the fermentation process according to the invention comprising silver nanoparticles, the probiotic strain belonging to the species of Lattobacillus or Bifidobacterium and a unicellular alga rich in omega-3 fatty acids, wherein said silver nanoparticles are characterized by an average size which varies within the range of 20 nm to 70 nm, preferably from 30 nm to 50 nm.

Furthermore, it is known from literature that various methods for the biosynthesis of nanoparticles totally change the composition of their crown (macromolecules, generally of a protein nature, which adhere to the nanoparticles) altering their properties, biological functions and bactericidal potential ([16],[17]). Crowns are therefore characteristic of specific nanoparticles, grown under specific conditions on specific growth media [18].

The invention also relates to the ternary mixture based on silver nanoparticles, probiotic and unicellular algae rich in omega-3 fatty acids that can be obtained by the process according to the invention for use as an anti-inflammatory and/or antiseptic agent.

An object of the present invention therefore also relates to a nutraceutical composition comprising the ternary mixture based on silver nanoparticles, probiotic and unicellular alga rich in omega-3 fatty acids that can be obtained by the process according to the invention as active ingredient, possibly together with one or more additional adjuvants and/or excipients acceptable from a nutraceutical point of view for use in the human or veterinary medical field.

A further object of the present invention also relates to a pharmaceutical composition comprising the ternary mixture based on silver nanoparticles, probiotic and unicellular alga rich in omega-3 fatty acids that can be obtained by the process according to the invention as active ingredient, possibly together with one or more additional adjuvants and/or pharmaceutically acceptable excipients for use in the human or veterinary medical field. The compositions according to the invention can be advantageously used, for example, in the treatment of cattle on intensive farms.

The ternary mixture based on silver nanoparticles, probiotic strain and unicellular alga rich in omega-3 fatty acids according to the invention can be advantageously used in therapeutic applications as an adjuvant for the treatment of acute or chronic inflammatory conditions, such as for example chronic inflammatory intestinal diseases (such as, for example, Crohn's disease and ulcerative colitis); autoimmune intestinal diseases, such as celiac disease or leaky gut syndrome; irritable bowel syndrome, gluten intolerance, metabolic syndrome, colitis; inflammatory vaginal diseases, such as, for example, vaginosis, vaginitis, vulvovaginal candidiasis with Candidiasis, yeast or fungal infections, such as Candida; atopic dermatitis, scarifications and insect bites.

According to a particularly preferred embodiment of the present invention, the ternary mixture based on silver nanoparticles, probiotic strain and unicellular alga rich in omega-3 fatty acids according to the invention can be used for the prevention and treatment of gastrointestinal infections, such as, for example, those mediated by *Helicobacter pylori.*

Said nutraceutical or pharmaceutical composition is preferably suitable for oral administration. Alternatively, the pharmaceutical compositions can be formulated for topical administration, such as for example by rectal or vaginal application.

Preferred forms of oral administration include the formulation of the above-mentioned compositions as granules, lyophilizates, solutions, suspensions, soft capsules, pills, tablets, mouthwashes.

Preferred forms of topical, rectal or vaginal administration comprise the formulation of the above compositions as creams, ointments, lotions, gels, ovules, douches, micro-enemas, suppositories.

Preferably, said nutraceutical composition is suitable for oral administration, in the form of lyophilizates, granules, solutions, suspensions or capsules.

According to an alternative embodiment, the pharmaceutical and nutraceutical compositions according to the invention can be used as an adjuvant for antibiotic therapy, as they allow lower quantities of antibiotic to be used. In particular, infections mediated by microorganisms selected from the group consisting of *Staphylococcus sp., Enterobacter sp., Clostridium sp., Acinetobacter sp., Yersiniasp., Pasturella sp., Pseudomonas aeruginosa, Klebsiella pneumoniae, Bacillus subtilis, Escherichia coli, Helicobacter pylori, Streptococcus sp., Micrococcus luteus, Chlamydia sp., Mycobacterium sp., Salmonella enterica Typhi;* yeasts selected from the group consisting of *Candida albicans, Candida tropicalise, Saccharomyces cerevisiae;* fungi selected from the group consisting of *Trichophyton rubrum, Aspergillus sp., Borrelia sp.,* can benefit from the use of nutraceutical or pharmaceutical compositions according to the invention in which the Ag can exert its antimicrobial action.

The present invention will now be described for illustrative but non-limitative purposes, according to a preferred embodiment, with particular reference to the attached figures, in which:

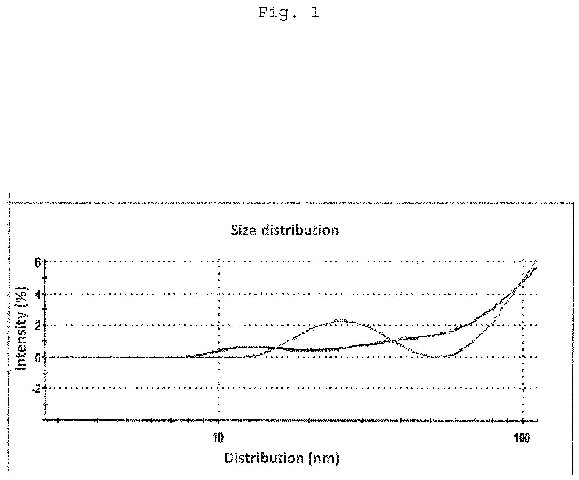

- Figure 1 shows the distribution of the peaks of nanoparticles separated by size (nm=nanometers, produced by *Lactobacillus reuteri* in the absence and in the presence of AgNO₃) by means of DLS analysis. After anaerobic growth of *Lactobacillus reuteri* in the absence of AGNO₃ (dark line), and in the presence of AGNO₃ (light line). The peak that appears at 26.21 ± 7.01 nanometers is specific of the condition with AGNO₃.

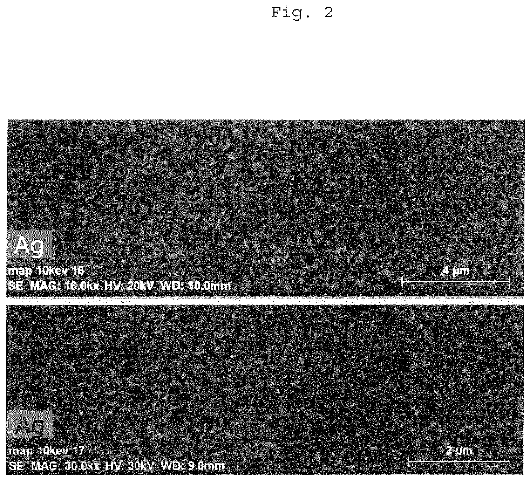

- Figure 2 shows the analysis of nanoparticles by means of electron microscopy associated with X-ray spectrometry. After aerobic growth (upper field) and anaerobic growth (lower field) of *Lactobacillus reuteri* in the presence of AGNO₃, the samples were dialyzed and fixed on an aluminum support for electron microscopy. The light dots represent silver ions aggregated in nanoparticles of the size expected from the DLS analysis.

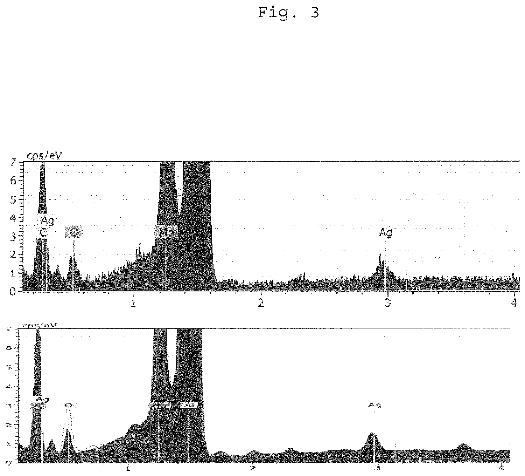

- Figure 3 shows the detail of the spectrum of the samples shown in Figure 2 through the Bruker Quantax EDS X-ray Microanalysis system. After aerobic growth (upper field) and anaerobic growth (lower field) of *Lactobacillus reuteri* in the presence of AGNO₃, the samples were dialyzed and fixed on an aluminum support for electron microscopy. The elements described in the box represent the most abundant of the coloured particles of Figure 2. Mg and Al are constituents of the support for electron microscopy, Carbon, Oxygen and Silver are the main constituents of the nanoparticles.

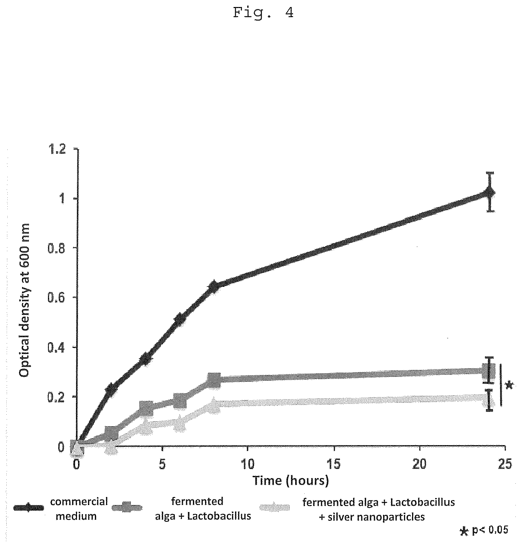

- Figure 4 shows the growth curve of the bacterium *E. coli* LF82 from 0 to 24 hours in a commercial growth medium (black line), in *Lactobacillus reuteri* with alga (dark grey line), in *Lactobacillus reuteri* with alga in the presence of silver nanoparticles (light grey line).

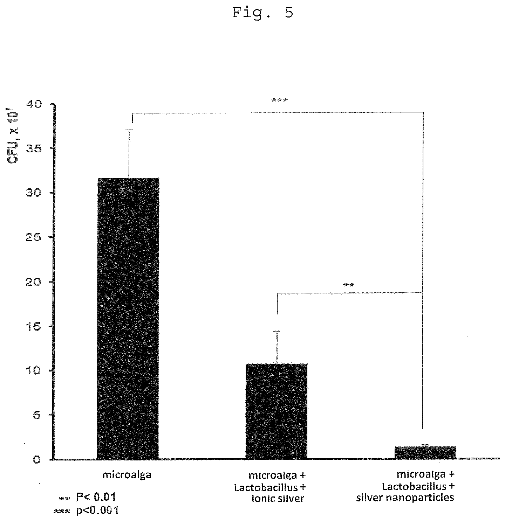

- Figure 5 shows the CFU count of *E.coli* LF82 after 24 hours of growth in medium with: 1) microalga (column 1); 2) microalga + *Lactobacillus reuteri* + silver in ionic form (column 2); 3) in microalga + *Lactobacillus reuteri* + silver nanoparticles (column 3).

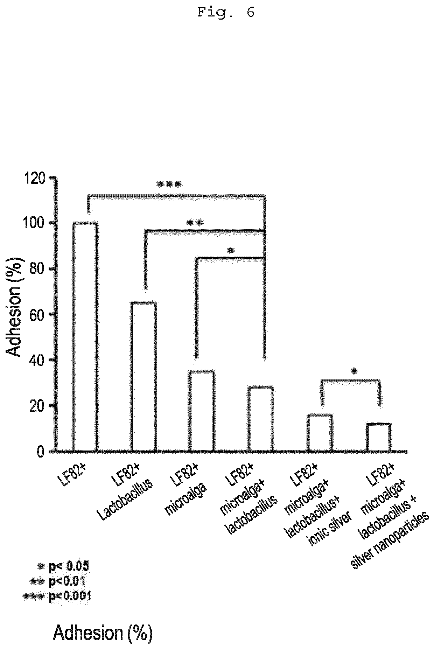

- Figure 6 shows the adhesion percentage of *E.coli* LF82 to CACO₂ after 3 hours of contact in growth medium for D-MEM eukaryotic cells. The number of CFUs of LF82 alone was set as 100%.

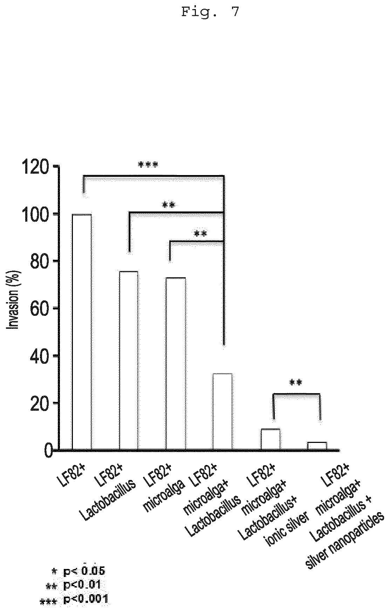

- Figure 7 shows the invasion percentage of LF82 with respect to CACO₂ after 3 hours of contact in a growth medium for D-MEM eukaryotic cells and subsequent washing with gentamicin. The number of CFUs of LF82 alone was set as 100%.

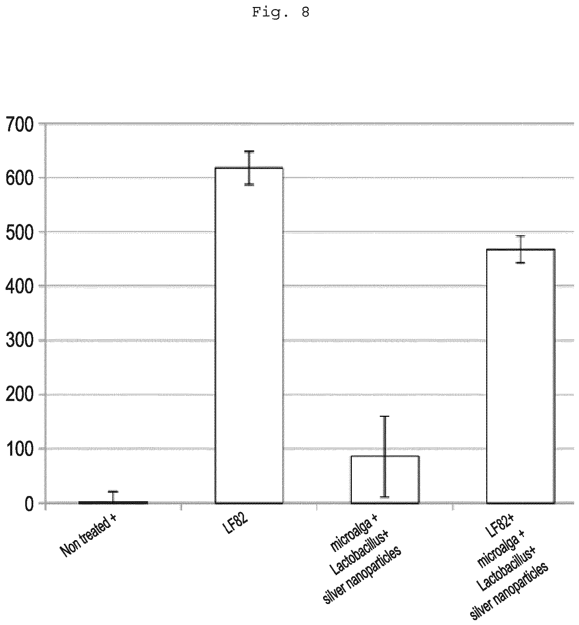

- Figure 8 shows the expression analysis of the interleukin 8 transcript (IL-8) in CACO₂ by RT-PCR after treatment with: 1) basal (column 1); 2) *E.coli* LF82 (column 2); 3) microalga, *Lactobacillus reuteri* and silver nanoparticles (column 3); 4) *E. coli* LF82, microalga, *Lactobacillus reuteri* and silver nanoparticles (column 4).

The following examples are now provided for a better illustration of the invention, which should be considered illustrative and non-limitative thereof.

### EXAMPLE 1: Process for the synthesis of silver nanoparticles

In order to obtain silver nanoparticles, lactobacilli or bifidobacteria (from 10⁵ to 10⁷ CFU/ml) were inoculated, dehydrated commercially available, in aqueous medium containing unicellular algae, of the genus *Isochrisys sp.* (or *Nannochloropsis sp.,* preferably *N. gaditana*) at a concentration of 30-40 g/L, to which silver nitrate (AgNO₃) was added at a concentration ranging from 2 to 5 µM as an inorganic donor of silver ions.

The cultivation was continued for 6 days by placing the flasks in a thermostatic cabinet at 37°C, under aerobic conditions (the strains belonging to the genus Lattobacillus are optional anaerobes) according to the following protocol:

### First day:

Preparation of the alga *Isochrysis sp.* or *Nannochloropsis gaditana:* 3.6 g of lyophilized alga in 100 ml of saline solution.

### Second day:

Preparation of solution A: *Lactobacillus reuteri:* 0.8 g of *Lactobacillus reuteri* DSM 17938 lyophilisate (BioGaia, Sweden), 1x10¹¹ CFU/ml, is re-suspended in 40 ml of alga in saline solution.

Solution B: 2 microliters of a 1 molar solution of AgNO₃ are added for each ml of solution A, to obtain a final concentration of 2 mM.

Solution B is left to ferment for 5 days at 37°C in an incubator, without agitation under aerobic or anaerobic conditions.

In the case of the use of strains belonging to the genus Bifidobacterium which are obligatory anaerobes, anaerobic conditions were used.

It was observed that the presence of AgNO₃ in the culture medium led to an initial slowdown in the growth of probiotic strains (first day), which, however, then followed a trend comparable to that in the absence of AgNO₃.

On the fifth day, the growth curve, until then in exponential phase, slowed down entering a plateau phase.

At the end of the process, and therefore the 5 days of cultivation, the neo-synthesized silver nanoparticles in the culture medium were identified and characterized by a double approach: Dynamic Light Scattering (DLS) analysis and electron microscopy scanning associated with X-ray spectrometry (EDS). The DLS analysis (see Figure 1) showed the presence of a specific peak in the sample grown in the presence of AgNO₃ of 26.21 ± 7.01 nanometers with respect to the sample grown without AgNO₃, suggesting that, even under these specific growth conditions as previously demonstrated in other conditions, the probiotic strains are capable of neutralizing the toxic effects of the silver ion turning it into nanoparticle. In order to make the presence of nanoparticles and the presence of a specific crown more certain, the DLS analyses were repeated by subjecting the samples to preventive washing in HCl and sonication to dissolve and disintegrate the organic matter of the crown and make the inorganic component more usable. (silver of nanoparticles). These experiments confirmed the presence of the peak previously observed and therefore of the metallic nucleus of the nanoparticle covered by a specific crown with unique characteristics.

The analysis by electron microscopy associated with X-ray spectrometry was carried out after repeated dialysis of the samples (in order to completely eliminate the single ions, both silver and others that could interfere with the method) to detect the presence of silver, not in ionic form, but of a nanoparticle having the dimensions observed by DLS. X-ray spectrometry confirmed the presence of silver nanoparticles both in samples grown under aerobic and anaerobic conditions (see Figure 2). The presence of silver in a constant quantity in the nanoparticles was confirmed, as the third most abundant element after carbon and oxygen (see Figure 3), through a completely automatic analysis software, questioned blindly.

The existence of silver nanoparticles synthesized from Lactobacilli or Bifidobacteria that grow, in both aerobic and anaerobic conditions, in green or blue microalgae was observed for the first time using the best technologies currently available.

### EXAMPLE 2:

*Study on the antibacterial and anti-inflammatory properties of the compositions obtained by the fermentation process according to the invention*

In the present study, in vitro experiments were carried out to evaluate the presence of a synergistic anti-inflammatory effect of the ternary mixture in addition to the antiseptic properties of the silver nanoparticles obtained after 5 days of cultivation according to the fermentation process of the invention.

For this purpose, a stabilized cell line (CACO₂) of intestinal epithelial cells originating from human colonic adenocarcinoma was used, now universally accepted as an *in vitro* intestinal epithelial barrier model.

### RESULTS

The experiments described above showed that exposure of the cells to the ternary mixture obtained with the fermentation process described: 1) inhibited the growth of a bacterial strain called LF82 belonging to the group of Escherichia coli invasive adhesives (AIEC). It has been shown that these bacteria can be positively selected in the inflamed intestine and that they are themselves responsible for triggering inflammation.

The following protocol was used for the cultivation of LF82:
First day: 1 single agar plate colony, sown the previous day, is inoculated in 5 ml of TSB and left to grow overnight at 37°C in an incubator, under mild agitation.
Second day: the bacterial culture is diluted in new growth medium until reaching the optical density (OD) read at 600 nm of 0.55.
Solution C: 1 ml of this culture OD₆₀₀ = 0.55 is centrifuged for 10 minutes at 3,500 rpm at 4°C, and then re-suspended in 0.5 ml of saline solution.

In particular, the results obtained showed, in a growth assay monitored by 600 nm optical densitometry, that the strain of *E. coli* LF82 grows much less in a medium consisting of alga and probiotic (red line) than in a standard medium (black line), but above all (green line) when nanoparticles are synthesized in the medium by lactobacilli.

This property, illustrated in the graph shown in Figure 4 in the first 24 hours of growth, is maintained and even improves in the following days. Figure 5 shows that at the end of the process, the AIEC LF82 is almost completely neutralized and that this effect is not due to its inability to grow in microalga but by the presence of the probiotic and especially of the nanoparticles synthesized by the same. There is, in fact, a statistically significant difference between both the colony forming units (CFUs) of LF82 in algal medium and algal medium + probiotic + nanoparticles (p<0.001), and among the CFUs in algal medium + probiotic and algal medium + probiotic + nanoparticles (p<0.01); 2) significantly reduced the adhesion and invasion capacity of the *E. coli* LF82 strain to a single layer of human intestinal cells, CACO₂. Adhesion and invasion of the intestinal mucosa cells is a function that in pseudo-pathogenic AIEC strains is particularly marked compared to the rest of the microbiota and is a preliminary condition for their pro-inflammatory activity [19].

The results of the adhesion test show that, taken individually, the lactobacilli and the microalga have the capacity of decreasing the number of AIEC LF82 that adhere to human intestinal cells (see Figure 6, columns 2 and 3); when they are pre-fermented and administered together, the adhesion percentage further decreases (see Figure 6, column 4); when ionic silver is added to the pre-fermented mixture of alga and lactobacilli, the adhesion percentage is even higher, due to the known antibacterial properties of ionic silver (see Figure 6, column 5); if, however, silver is added before the fermentation process and is then converted into nanoparticles, this effect is maximum and statistically significant (p<0.05), demonstrating that the fermentation process with the consequent production of nanoparticles increases the inhibition effectiveness of AIEC adherence in a synergistic and specific manner (see Figure 6, column 6).

The same applies to the *in vitro* invasion test, there is in fact a statistically significant difference between the invasion percentage of the *E.coli* LF82 strain in CACO₂ cells exposed to microalga + lactobacillus + ionic silver and microalga + lactobacillus + silver nanoparticles (p<0.01) (see Figure 7). 3) decreased the basal levels of interleukin 8 (IL-8) caused by exposure of CACO₂ cells to the *E. coli* LF82 strain. It should be noted that the treatment with the combination Lactobacillus + microalga + silver nanoparticles in the absence of LF82 does not significantly influence this level of IL-8 (see Figure 8, column 3), whereas if effected in a condition of contemporary exposure to LF82 it has the power to reduce the level of IL-8 by about 25% (see Figure 8, column 4).

In conclusion, it is shown that: i) microalgae represent an excellent substrate for the growth under aerobic/anaerobic conditions of probiotic strains belonging to the genus Lactobacillus sp.; ii) if the algae contain an adequate percentage of omega-3 fatty acids, the formulation has an added value as a supplement for human use; iii) if ionic silver is added to the culture medium, the probiotic strains under these conditions are able to synthesize silver nanoparticles; 4) the ternary formulation based on microalgae, Lactobacilli and biogenic silver nanoparticles has marked anti-inflammatory and antiseptic properties.

The same results were obtained using a Bifidobacteria strain (data not shown).

### BIBLIOGRAPHY

[1] Iravani S. Int. Res. Notices 2014.
[2] Elbeshehy EK et al., Frontiers in Microbiology 2015; 6:453.
[3] Kalimuthu K. et al. Colloids Surf 2008; 65:150-153.
[4] Kalishwaralal K. et al. 2008, Mater. Lett. 2008, 62:4411-3.
[5] Saifuddin N. et al. Journal of Chemistry 2009, 6:61-70.
[6] Pantidos N. and Horsfall L. J. Nanomed Nanotechnol 2014; 5:233.
[7] Jannathul Firdhouse M. and Lalitha P. 2015; Volume 2015, Article ID 829526.
[8] Daohu S et al. Acta Scientiae Circumstantiae 2006; 26, 1107-1110.
[9] Garmasheva I. et al. BioImpacts 2016; 6: 219-223.
[10] Ranganath E., Vandana R., Afreen B. IOSR J. Pharmacy 2012; 2:237-241.
[11] Mohseniazar M et al. Bioimpacts 2011; 1:149-152.
[12] Zhang M. et al. Biofouling 2014, 30:347-57.
[13] Arokiyaraj S. et al. Artif. Cells Nanomed Biotechnol. 2017; 45:372-379.
[14] Talukder RM et al. Biochem. Eng. J. 2012; 68:109-113.
[15] Nguyen CM et al. Riores. Tech. 2012; 10: 552-559.
[16] Juling S et al. J Proteome Res. 2017 Nov 3;16(11):4020-4034.
[17] Ban DK, Paul S. Colloids Surf B Biointerfaces. 2016 Oct 1;146:577-84.
[18] Miclaus et al, Nano Lett 2014;1:2086-93.
[19] Darfeuille-Michaud A et al. Gastroenterology 2004; 127:412-21.

## Claims

1. A process for the synthesis of a ternary mixture of silver nanoparticles, probiotic and unicellular algae rich in omega-3 fatty acids comprising the following steps:
a) inoculation of a probiotic strain belonging to the genus *Lactobacillus sp.* or *Bifidobacterium sp.* at a concentration ranging from 10⁵ to 10⁷ CFU/ml in an aqueous medium comprising unicellular algae rich in omega-3 fatty acids and an inorganic donor of silver ions;
b) cultivation at 37°C under aerobic or anaerobic conditions for 5-6 days.

2. The process according to claim 1, further comprising step c) for the lyophilization of the mixture obtained in step b).

3. The process according to any of the previous claims, wherein said unicellular algae are present in the culture medium at a concentration of 30-40 g/L.

4. The process according to claim 3, wherein said unicellular algae rich in omega-3 acids are green or blue microalgae selected from the group consisting of *Pavlova sp., Isochrisys sp., Nannochloropsis sp., Porphyridium sp., Cryptomonas sp., Rhodomonas sp.,* and *Tetraselmis sp.,* preferably *Nannochloropsis gaditana.*

5. The process according to any of the previous claims, wherein said inorganic donor of silver ions is added to the culture medium at a concentration ranging from 2 to 5 µM.

6. The process according to claim 5, wherein the inorganic donor of silver ions is AgNO₃.

7. The process according to any of the previous claims, wherein the probiotic strain belonging to the genus *Lactobacillus sp.* is selected from the group consisting of *L. reuteri, L. brevis, L. buchneri, L. fermentum* or *L. casei, L. curvatus, L. plantarum, L. sakei, L. paracasei,* preferably *L.reuteri.*

8. The process according to any of the previous claims, wherein the probiotic strain belonging to the genus *Bifidobacterium sp.* is selected from the group consisting of *B. lactis* or *B. brevis.*

9. A ternary mixture based on silver nanoparticles, probiotic and omega-3 fatty acids that can be obtained by the fermentation process according to any of claims 1-8, wherein the silver nanoparticles are **characterized by** an average size which varies within the range of 20 nm to 70 nm, preferably from 30 nm to 50 nm.

10. The ternary mixture according to claim 9, for use in the human or veterinary medical field as an anti-inflammatory and/or antiseptic agent.

11. The ternary mixture according to claim 10, for the treatment of acute or chronic inflammatory diseases, intestinal or vaginal, selected from the group consisting of Crohn's disease, ulcerative colitis, celiac disease, leaky gut syndrome, irritable bowel syndrome, gluten intolerance, metabolic syndrome, colitis, vaginosis, vaginitis, vaginal yeast or fungal infections, such as Candida, dermatitis, scarifications, insect bites.

12. The ternary mixture according to claim 10, for use as an antiseptic in the treatment of infections mediated by microorganisms selected from the group consisting of *Staphylococcus sp., Enterobacter sp., Clostridium sp., Acinetobacter sp., Yersiniasp., Pasturella sp., Pseudomonas aeruginosa, Klebsiella pneumoniae, Bacillus subtills, Helicobacter pylori, Escherichia coli, Streptococcus sp., Micrococcus luteus, Chlamydia sp., Mycobacterium sp., Salmonella enterica Typhi,* yeasts selected from the group consisting of *Candida albicans, Candida tropicalise, Saccharomyces cerevisiae;* fungi selected from the group consisting of *Trichophyton rubrum, Aspergillus sp., Borrelia sp.*

13. A nutraceutical composition comprising the ternary mixture according to claim 9 as active ingredient, optionally together with one or more further adjuvants and/or excipients acceptable from a nutraceutical point of view.

14. A pharmaceutical composition comprising the ternary mixture according to claim 9 as active ingredient, optionally together with one or more further adjuvants and/or excipients acceptable from a pharmaceutical point of view for use in the human or veterinary medical field.

15. The nutraceutical composition according to claim 13 or the pharmaceutical composition according to claim 14, suitable for oral administration.

16. The pharmaceutical composition according to claim 14, suitable for topical administration, preferably rectal or vaginal.

## Patentansprüche

1. Verfahren zur Synthese eines ternären Gemisches aus Silbernanopartikeln, probiotischen und einzelligen Algen, die reich an Omega-3-Fettsäuren sind, umfassend folgende Schritte:
a) Inokulation eines probiotischen Stammes, gehörend zur Art *Lactobacillus sp.* oder *Bifidobacterium sp.* in einer Konzentration im Bereich von 10⁵ bis 10⁷ KbE/ml, in einem wässrigen Medium umfassend einzellige Algen, die reich an Omega-3-Fettsäuren sind, und einen anorganischen Silber-Ionen-Donator;
b) Kultivierung bei 37°C unter aeroben oder anaeroben Bedingungen 5-6 Tage lang.

2. Verfahren nach Anspruch 1, ferner umfassend Schritt c) zur Gefriertrocknung der in Schritt b) erhaltenen Mischung.

3. Verfahren nach irgendeinem der vorhergehenden Ansprüche, wobei die einzelligen Algen in dem Kulturmedium in einer Konzentration von 30 bis 40 g/L vorhanden sind.

4. Verfahren nach Anspruch 3, wobei die an Omega-3-Säuren reichen einzelligen Algen grüne oder blaue Mikroalgen sind, ausgewählt aus der Gruppe bestehend aus *Pavlova sp., Isochrisys sp., Nannochloropsis sp., Porphyridium sp., Cryptomonas sp., Rhodomonas sp.,* und *Tetraselmis sp.,* vorzugsweise *Nannochloropsis gaditana.*

5. Verfahren nach irgendeinem der vorhergehenden Ansprüche, wobei der anorganische Silber-Ionen-Donator dem Kulturmedium in einer Konzentration im Bereich von 2 bis 5 µM zugegeben wird.

6. Verfahren nach Anspruch 5, wobei der anorganische Silber-Ionen-Donator AgNO₃ ist.

7. Verfahren nach irgendeinem der vorhergehenden Ansprüche, wobei der zur Art *Lactobacillus sp.* gehörende probiotische Stamm ausgewählt ist aus der Gruppe bestehend aus *L. reuteri, L. brevis, L. buchneri, L. fermentum oder L. casei, L. curvatus, L. plantarum, L. sakei, L. paracasei, vorzugsweise L. reuteri.*

8. Verfahren nach irgendeinem der vorhergehenden Ansprüche, wobei der zur Art *Bifidobacterium sp.* gehörende probiotische Stamm ausgewählt ist aus der Gruppe bestehend aus *B. lactis* oder *B. brevis.*

9. Ternäre Mischung auf der Basis von Silbernanopartikeln, probiotischen und Omega-3-Fettsäuren, die durch das Fermentationsverfahren nach irgendeinem der Ansprüche von 1 bis 8 erhalten werden kann, wobei die Silbernanopartikel durch eine durchschnittliche Größe gekennzeichnet sind, die innerhalb des Bereichs von 20 nm bis 70 nm, vorzugsweise von 30 nm bis 50 nm variiert.

10. Ternäre Mischung nach Anspruch 9 zur Verwendung im human- oder veterinärmedizinischen Bereich als entzündungshemmendes und/oder antiseptisches Mittel.

11. Ternäre Mischung nach Anspruch 10 zur Behandlung von akuten oder chronischen entzündlichen Erkrankungen des Darms oder der Vagina, ausgewählt aus der Gruppe bestehend aus Morbus Crohn, Colitis ulcerosa, Zöliakie, Leaky-Gut-Syndrom, Reizdarmsyndrom, Glutenintoleranz, metabolischem Syndrom, Kolitis, Vaginose, Vaginitis, Vaginalhefe oder Pilzinfektionen wie Candida, Dermatitis, Skarifikationen, Insektenstiche.

12. Ternäre Mischung nach Anspruch 10 zur Verwendung als Antiseptikum bei der Behandlung von Infektionen, die durch Mikroorganismen vermittelt werden, ausgewählt aus der Gruppe bestehend aus *Staphylococcus sp., Enterobacter sp., Clostridium sp., Acinetobacter sp., Yersinia sp., Pasturella sp.,. Pseudomonas aeruginosa, Klebsiella pneumoniae, Bacillus subtills, Helicobacter pylori, Escherichia coli, Streptococcus sp., Micrococcus luteus, Chlamydia sp., Mycobacterium sp., Salmonella enterica Typhi,* Hefen, ausgewählt aus der Gruppe bestehend aus *Candida albicans, Candida tropicalise, Saccharomyces cerevisiae;* Pilze ausgewählt aus der Gruppe bestehend aus *Trichophyton rubrum, Aspergillus sp., Borrelia sp.*

13. Nutrazeutische Zusammensetzung, umfassend die ternäre Mischung nach Anspruch 9 als Wirkstoff, gegebenenfalls zusammen mit einem oder mehreren weiteren Adjuvantien und/oder Hilfsstoffen, die aus nutrazeutischer Sicht akzeptabel sind.

14. Pharmazeutische Zusammensetzung, umfassend die ternäre Mischung nach Anspruch 9 als Wirkstoff, gegebenenfalls zusammen mit einem oder mehreren weiteren Adjuvantien und/oder Hilfsstoffen, die aus pharmazeutischer Sicht zur Verwendung im Bereich der Human- oder Veterinärmedizin akzeptabel sind.

15. Nutrazeutische Zusammensetzung nach Anspruch 13 oder pharmazeutische Zusammensetzung nach Anspruch 14, die zur oralen Verabreichung geeignet ist.

16. Pharmazeutische Zusammensetzung nach Anspruch 14, die zur topischen, vorzugsweise rektalen oder vaginalen Verabreichung geeignet ist.

## Revendications

1. Procédé pour la synthèse d'un mélange ternaire de nanoparticules d'argent, probiotiques et algues unicellulaires riches en acides gras oméga-3 comprenant les étapes suivantes :
a) inoculation d'une souche probiotique appartenant au genre *Lactobacillus sp.* ou *Bifidobacterium sp.* à une concentration allant de 10⁵ à 10⁷ CPU/ml dans un milieu aqueux comprenant des algues unicellulaires riches en acides gras oméga-3 et un donneur inorganique d'ions argent ;
b) culture à 37°C sous des conditions aérobies et anaérobies pendant 5-6 jours.

2. Procédé selon la revendication 1, comprenant en outre une étape c) pour la lyophilisation du mélange obtenu à l'étape b).

3. Procédé selon l'une quelconque des revendications précédentes, dans lequel lesdites algues unicellulaires sont présentes dans le milieu de culture à une concentration de 30-40 g/L.

4. Procédé selon la revendication 3, dans lequel lesdites algues unicellulaires riches en acides gras oméga-3 sont des microalgues vertes ou bleues sélectionnées dans le groupe constitué de *Pavlova sp., Isochrisys sp., Nannochloropsis sp., Porphyridium sp., Cryptomonas sp., Rhodomonas sp.,* et *Tetraselmis sp.,* de préférence *Nannochloropsis gaditana.*

5. Procédé selon l'une quelconque des revendications précédentes, dans lequel ledit donneur inorganique d'ions argent est rajouté au milieu de culture à une concentration allant de 2 à 5 µM.

6. Procédé selon la revendication 5, dans lequel le donneur inorganique d'ions argent est AgNO₃.

7. Procédé selon l'une quelconque des revendications précédentes, dans lequel la souche probiotique appartenant au genre *Lactobacillus sp.* est sélectionnée dans le groupe constitué de *L. reuteri, L. brevis, L. buchneri, L. fermentum* ou *L. casei, L. curvatus, L. plantarum, L. sakei, L. paracasei,* de préférence *L. reuteri.*

8. Procédé selon l'une quelconque des revendications précédentes, dans lequel la souche probiotique appartenant au genre *Bifidobacterium sp.* est sélectionnée dans le groupe constitué de *B. lactis* ou *B. brevis.*

9. Mélange ternaire basé sur des nanoparticules d'argent, probiotiques et acides gras oméga-3 qui peut être obtenu par le procédé de fermentation selon l'une quelconque des revendications 1-8, dans lequel les nanoparticules d'argent sont **caractérisées par** une dimension moyenne qui varie à l'intérieur de la plage de 20 nm à 70 nm, de préférence de 30 nm à 50 nm.

10. Mélange ternaire selon la revendication 9, pour une utilisation dans le domaine de la médecine humaine ou vétérinaire en tant qu'agent anti-inflammatoire et/ou antiseptique.

11. Mélange ternaire selon la revendication 10, pour le traitement de maladies inflammatoires aiguës ou chroniques, intestinales ou vaginales, sélectionnées dans le groupe constitué de maladie de Crohn, colite ulcéreuse, maladie cœliaque, syndrome d'hyperperméabilité intestinale, syndrome de l'intestin irritable, intolérance au gluten, syndrome métabolique, colite, vaginose, vaginite, mycose vaginale ou infections fongiques, telles que candidose, dermatite, scarifications, piqûres d'insecte.

12. Mélange ternaire selon la revendication 10, pour une utilisation en tant qu'antiseptique dans le traitement d'infections provoquées par des micro-organismes sélectionnés dans le groupe constitué de *Staphylococcus sp., Enterobacter sp., Clostridium sp., Acinetobacter sp., Yersinia sp., Pasturella sp., Pseudomonas aeruginosa, Klebsiella pneumoniae, Bacillus subtills, Helicobacter pylori, Escherichia coli, Streptococcus sp., Micrococcus luteus, Chlamydia sp., Mycobacterium sp., Salmonella enterica Typhi,* levures sélectionnées dans le groupe constitué de *Candida albicans, Candida tropicalise, Saccharomyces cerevisiae* ; champignons sélectionnés dans le groupe constitué de *Trichophyton rubrum, Aspergillus sp., Borrelia sp.*

13. Composition nutraceutique comprenant le mélange ternaire selon la reveortondication 9 en tant que substance active, optionnellement avec un ou plusieurs autres adjuvants et/ou excipients acceptables d'un point de vue nutraceutique.

14. Composition pharmaceutique comprenant le mélange ternaire selon la revendication 9 en tant que substance active, optionnellement avec un ou plusieurs autres adjuvants et/ou excipients acceptables d'un point de vue pharmaceutique pour une utilisation dans le domaine de la médecine humaine ou vétérinaire.

15. Composition nutraceutique selon la revendication 13 ou composition pharmaceutique selon la revendication 14 adaptée pour une administration orale.

16. Composition pharmaceutique selon la revendication 14, adaptée pour une administration topique, de préférence rectale ou vaginale.
